# EUROPÄISCHE PATENTANMELDUNG

(11) **EP 4 840 488 A1**
(43) Veröffentlichungstag der Anmeldung: **18.11.2026**
(21) Anmeldenummer: 26178251.0
(22) Anmeldetag: 12.05.2026
(51) Int. Cl.: A61M 5/315, A61M 5/28

(54) **SPRITZENLEHRE FÜR EINE SPRITZE UND SPRITZENSYSTEM**

(30) Priorität: 15.05.2025 DE 102025118888
(71) Anmelder: Albomed GmbH, 90592 Schwarzenbruck (DE)
(72) Erfinder: Reese, Sven, 90592 Schwarzenbruck (DE)
(74) Vertreter: Lichti - Patentanwälte Partnerschaft mbB

(57) **Zusammenfassung**

Die Erfindung betrifft eine Spritzenlehre für eine Spritze mit einem in einem Spritzenzylinder längsverschieblich angeordneten Spritzenkolben zum Einstellen eines vorgegebenen Injektionsvolumens. Die Spritzenlehre weist einen Verbindungsbereich mit mindestens zwei Verbindungsabschnitten auf, wobei die Verbindungsabschnitte jeweils ein Befestigungsmittel zum zerstörungsfrei lösbaren Aufnehmen der Spritzenlehre mit der Spritze aufweisen. Die Befestigungsmittel erstrecken sich parallel zu der Längsrichtung der Spritze erstrecken und die Verbindungsabschnitte sind senkrecht zu der Erstreckungsrichtung der Befestigungsmittel versetzt zueinander angeordnet.

## Beschreibung

Die Erfindung betrifft eine Spritzenlehre für eine Spritze und ein Spritzensystem zum Einstellen eines vorgegebenen Injektionsvolumens.

Aus dem Stand der Technik sind Dosierhilfen für Spritzen bekannt, die zum Einstellen eines vorgegebenen Injektionsvolumens mehrere, axial hintereinander angeordnete und über Sollbruchstellen miteinander verbundene Distanzstücke aufweisen, um eine definierte Anschlagsposition für die Bewegung eines Spritzenkolbens in einem Spritzenzylinder auszubilden. Die bekannten Dosierhilfen können in einer bestimmten Konfiguration stets nur eine einzige Anschlagsposition vorsehen.

Es ist daher die Aufgabe der Erfindung, diese Nachteile zu beseitigen und insbesondere eine Spritzenlehre zu entwickeln, mit der leicht mehrere vordefinierte Injektionsvolumina eingestellt werden können.

Die Aufgabe wird gelöst mit einer Spritzenlehre nach Anspruch 1. Dabei handelt es sich um eine Spritzenlehre für eine Spritze mit einem in einem Spritzenzylinder längsverschieblich angeordneten Spritzenkolben zum Einstellen eines vorgegebenen Injektionsvolumens, wobei die Spritzenlehre einen Verbindungsbereich mit mindestens zwei Verbindungsabschnitten aufweist, wobei die Verbindungsabschnitte jeweils ein Befestigungsmittel zum Aufnehmen der Spritzenlehre mit der Spritze aufweisen, wobei sich die Befestigungsmittel parallel zu der Längsrichtung der Spritze erstrecken und wobei die Verbindungsabschnitte senkrecht zu der Erstreckungsrichtung der Befestigungsmittel versetzt zueinander angeordnet sind.

Weiterhin wird die Aufgabe gelöst mit einem Spritzensystem nach Anspruch 8, das eine Spritze und eine mit der Spritze verbindbaren, insbesondere mit der Spritze verbundene, erfindungsgemäße Spritzenlehre aufweist.

Die Erfindung basiert auf der Grundüberlegung, dass durch die senkrecht zu der Erstreckungsrichtung der Befestigungsmittel versetzt zueinander angeordneten Verbindungsabschnitte eine einfache Möglichkeit der Auswahl von mehreren vordefinierten Injektionsvolumina gegeben ist, denn der Benutzer kann sich durch die einfache und schnelle Auswahl eines Verbindungsabschnitts, mit dem die Spritze verbunden werden kann, für ein vordefiniertes Injektionsvolumen entscheiden es entfällt insbesondere die Notwendigkeit, für mehrere mögliche Injektionsvolumina Sollbruchstellen auszubilden, so dass die Spritzenlehre nach dem Einsatz mit einer bestimmten Spritze ohne weiteres für eine weitere Spritze wiederverwertbar ist.

Das Befestigungsmittel, insbesondere sämtliche Befestigungsmittel, können zum zerstörungsfrei lösbaren Aufnehmen der Spritzenlehre mit der Spritze ausgestaltet sein. Das Befestigungsmittel kann insofern als Fixationsmittel für die Spritze ausgebildet sein, das beispielsweise für eine dauerhafte Verbindung der Spritzenlehre mit der Spritze ausgelegt sein kann. Das Fixationsmittel kann zum Halten der Spritze ausgebildet sein.

In einer Weiterbildung der Erfindung kann die Spritzenlehre für den Einmalgebrauch ausgebildet sein. In einer alternativen Ausgestaltung kann die Spritzenlehre wiederverwendbar sein, was durch die leichte Verbindbarkeit der Spritze mit der Spritzenlehre und/oder die leichte Lösbarkeit der Spritze mit der Spritzenlehre erleichtert wird.

Der Abstand der Befestigungsmittel kann mindestens 2 mm und/oder höchstens 50 mm betragen. Der Abstand der Befestigungsmittel kann abhängig von dem Typ der Spritze sein. Die Spritzenlehre kann sterilisiert sein.

Vorzugsweise sind die Verbindungsabschnitte zum Aufnehmen der Spritze mit dem Spritzenkolben, insbesondere mit der Spritzenkolbenstange, höchst vorzugsweise mit der Spritzenkolbenstange, proximal des Spritzenzylinders ausgebildet. Die Verbindungsabschnitte können als Arretierungen für die Bewegung des Spritzenkolbens ausgebildet sein, um dessen Hub zu begrenzen, wenn die Spritze von der Spritzenlehre aufgenommen ist. Damit kann auch die Spritzenlehre insgesamt als Arretierung für die Längsbewegung des Spritzenkolbens ausgebildet sein.

Im Sinne der Erfindung weist eine axiale Richtung parallel zu der Längserstreckung der Befestigungsmittel, damit auch parallel zu der Längserstreckung der Spritze, und eine laterale Richtung steht senkrecht zu der axialen Richtung. Insofern sind die Befestigungsmittel der Spritzenlehre lateral versetzt zueinander angeordnet.

Zur Aufnahme der Spritze von der Spritzenlehre kann sich die Spritze zumindest bereichsweise in dem Befestigungsmittel befinden. Das Befestigungsmittel kann zur Aufnahme der Spritze unter enger Passung ausgebildet sein.

Die Toleranz zwischen dem Befestigungsmittel der Spritzenlehre und der Spritze im aufgenommenen Zustand kann abhängig von der Spritze, insbesondere von deren Modell und/oder von deren Spritzenkolben und/oder von dessen Schubstange sein. Die Toleranz kann mindestens 0,2 mm und/oder höchstens 2 mm betragen. Der Verbindungsabschnitt der Spritzenlehre kann zum Aufnehmen der Spritze unter enger Passung ausgebildet sein.

Vorzugsweise weist das Befestigungsmittel, insbesondere sämtliche Befestigungsmittel, zwei zueinander beabstandet angeordnete und/oder parallel zueinander ausgerichtete Vorsprünge auf, wobei mindestens einer der Vorsprünge nach unten ausgerichtet sein ich kann. In einer Weiterbildung der Erfindung kann vorgesehen sein, dass der mindestens eine Vorsprung im Querschnitt zumindest bereichsweise teilkreisförmig, insbesondere halbkreisförmig ausgebildet ist. Der Vorsprung kann sich senkrecht zu der Versatzrichtung des ihm zugeordneten Verbindungsabschnitts und/oder senkrecht zu der Längserstreckung des ihm zugeordneten Befestigungsmittels, insbesondere nach unten, erstrecken. Zwischen zwei Vorsprüngen eines Befestigungsmittels kann ein U-förmiger Zwischenbereich mit einem abschnittsweise halbkreisförmigen Querschnitt ausgebildet sein, der zur Anlage mit der Spritze ausgebildet sein kann. Zur Ermöglichung einer sicheren Aufnahme der Spritze, insbesondere von deren Spritzenzylinder, können sich die Vorsprünge über mindestens die Hälfte des Durchmessers der Spritze erstrecken, insbesondere über mehr als ¾ des Durchmessers der Spritze.

Vorzugsweise ist jedem Verbindungsabschnitt mindestens eine beispielsweise numerische oder nicht-numerische Information auf der Oberfläche der Spritzenlehre zur leichteren Unterscheidbarkeit der Verbindungsabschnitte zugeordnet. Dadurch kann der Benutzer einfach den für den jeweiligen Einsatzzweck idealen Verbindungsabschnitt zum Einstellen des vordefinierten Injektionsvolumens auswählen. Zu diesem Zweck kann die Information in Form einer Vertiefung oder einer Auswölbung ausgebildet sein, um die Information haptisch erfassen zu können, ohne dass der Benutzer insbesondere während oder zur Vorbereitung eines chirurgischen Eingriffs zu der Schieblehre blicken muss.

Vorzugsweise sind mindestens drei, insbesondere genau drei, mindestens vier, insbesondere genau vier, oder mindestens fünf, insbesondere genau fünf, Verbindungsabschnitte ausgebildet, die insbesondere sämtlich parallel zueinander ausgerichtet sein können und/oder die verschiedene Längen aufweisen können. In diesen Fällen entsprechen die Verbindungsabschnitte aufgrund ihrer unterschiedlichen Längen unterschiedlichen vordefinierten Injektionsvolumina. Es entspricht einem Grundgedanken der Erfindung, dass das mittels der erfindungsgemäßen Spritzenlehre einstellbare vordefinierte Injektionsvolumen maßgeblich von dem verbleibenden restlichen Hub der Spritze und dem Durchmesser des Spritzenzylinders abhängt, wenn der Spritzenkolben in Anlage mit der Spritzenlehre gelangt. Da der Durchmesser der Spritze fest vorgegeben ist, kann das einzustellende Injektionsvolumen einfach und schnell durch den verbleibenden restlichen Hub der Spritze einstellbar sein. Dies wird durch die unterschiedlich langen Verbindungsabschnitte ermöglicht. Mit anderen Worten können die Längen der Verbindungsabschnitte die Einstellung des zu applizierenden, vordefinierten Injektionsvolumens eines Injektionsmittels über die Festlegung des verbleibenden Spritzenhubes ermöglichen. Insofern kann der Verbindungsabschnitt insbesondere aufgrund seiner Länge dem diesen zugeordneten Injektionsvolumen entsprechen. In einer Weiterbildung der Erfindung weist die Spritzenlehre zwischen zwei und fünf Verbindungsabschnitte auf.

In einer Weiterbildung der Erfindung können die Längen der Verbindungsabschnitte den Längen der diesen jeweils zugeordneten Befestigungsmitteln entsprechen, wobei beispielsweise die Quotienten aus der Länge eines Verbindungsabschnitts mit der Länge des diesem zugeordneten Befestigungsmittel für mindestens zwei Verbindungsabschnitte, insbesondere für sämtliche Verbindungsabschnitte, im Wesentlichen gleich sein. Vorzugsweise ist die Länge von einem einem ersten Verbindungsabschnitt zugeordneten Befestigungsmittel verschieden von der Länge des Befestigungsmittels, das einem zweiten Verbindungsabschnitt, insbesondere mit einer von der Länge des ersten Verbindungsabschnitts verschiedenen Länge, zugeordnet ist.

In einer Weiterbildung der Erfindung kann vorgesehen sein, dass zwischen mindestens zwei Längen der Verbindungsabschnitte, insbesondere zwischen sämtlichen Längen der Verbindungsabschnitte, ein polynomialer Zusammenhang besteht, so dass sich die Längen der Verbindungsabschnitte durch eine Polynomialgleichung beschreiben lassen können. In einer Weiterbildung der Erfindung können sich die Längen der Verbindungsabschnitte durch eine Potenzfunktion beschreiben lassen. Insbesondere besteht zwischen mindestens zwei Längen der Verbindungsabschnitte, insbesondere zwischen sämtlichen Längen der Verbindungsabschnitte, ein linearer Zusammenhang, so dass sich die Längen der Verbindungsabschnitte durch eine lineare Gleichung beschreiben lassen können. Weiterbildungen der Erfindung können vorsehen, dass zwischen mindestens zwei Längen der Verbindungsabschnitte, insbesondere zwischen sämtlichen Längen der Verbindungsabschnitte, ein nichtlinearer Zusammenhang besteht, so dass sich die Längen der Verbindungsabschnitte durch eine nichtlineare Gleichung beschreiben lassen können. Insbesondere besteht zwischen mindestens zwei Längen der Verbindungsabschnitte, insbesondere zwischen sämtlichen Längen der Verbindungsabschnitte, ein quadratischer oder kubischer Zusammenhang. In einer Weiterbildung der Erfindung kann vorgesehen sein, dass zwischen mindestens zwei Längen der Verbindungsabschnitte, insbesondere zwischen sämtlichen Längen der Verbindungsabschnitte, ein logarithmischer Zusammenhang besteht, so dass sich die Längen der Verbindungsabschnitte durch eine logarithmische Funktion beschreiben lassen können.

Vorzugsweise ist zwischen zwei Verbindungsabschnitten, insbesondere zwischen unmittelbar zueinander benachbarten Verbindungsabschnitten, ein Übergang mit einer insbesondere abgerundeten Stufe ausgebildet, um die Verbindungsabschnitte leichter voneinander unterscheiden zu können. In einer Weiterbildung der Erfindung kann vorgesehen sein, dass die Stufen ausschließlich auf der proximalen Stirnseite der Spritzenlehre ausgebildet sind. Dadurch wird gewährleistet, dass der Benutzer die Spritzenlehre stets in der korrekten Ausrichtung hält. Die distale Stirnseite der Spritzenlehre kann eben ausgebildet sein. Im Sinne der Erfindung weist eine proximale Richtung zu dem Benutzer der Spritzenlehre und eine distale Richtung zu dem Patienten. Entsprechend ist eine von proximal nach distal oder umgekehrt weisende Richtung eine axiale oder Längsrichtung.

Vorzugsweise ist benachbart zu dem Verbindungsbereich ein Griffbereich ausgebildet, wobei der Griffbereich insbesondere benachbart zu dem Verbindungsabschnitt mit der größten Längserstreckung, insbesondere unmittelbar benachbart daneben, angeordnet ist. Dadurch kann Spritzenlehre leicht von dem Benutzer ergriffen und gehalten werden, insbesondere in der korrekten Ausrichtung. Der Griffbereich kann eine insbesondere kreisförmige Vertiefung aufweisen, die als Griffmulde ausgebildet sein kann. Die Griffmulde kann auf der Oberseite und/oder auf der Unterseite des Griffbereichs angeordnet sein.

In einer Weiterbildung der Erfindung kann vorgesehen sein, dass die Spritze mit einem Medikament für die Netzhaut zur Behandlung von Erkrankungen vorgefüllt ist.

Der Spritzenkolben kann an einem proximalen Endbereich eine laterale, insbesondere kreisförmige Verbreiterung aufweisen, die in Anlage mit der Spritzenlehre, insbesondere mit deren proximaler Stirnseite, bringbar ist. Der Spritzenzylinder kann an einem proximalen Endbereich eine insbesondere kreisförmige, laterale Verbreiterung aufweisen, die in Anlage mit der Spritzenlehre, insbesondere mit deren distaler Stirnseite bringbar ist. Die Spritzenlehre kann axial zwischen dem Spritzenkolben und dem Spritzenzylinder anordenbar sein.

Der Spritzenzylinder kann zwei einander gegenüber angeordnete, laterale Vorsprünge aufweisen, mit denen die Spritze leichter ergriffen werden kann und die als Widerlager für den Benutzer während der Längsbewegung des Spritzenkolbens in dem Spritzenzylinder dienen können. Die Länge der Verbindungsabschnitte der Spritzenlehre können auf den Durchmesser der Spritze, insbesondere auf den Durchmesser des Spritzenzylinders abgestimmt sein, damit mittels der Spritzenlehre ein vordefiniertes Injektionsvolumen der Spritze einfach einstellbar ist.

Vorzugsweise ist die Spritzenlehre, insbesondere das Spritzensystem, zur Verwendung bei einem chirurgischen Eingriff an oder in einem Auge vorgesehen, wobei die Spritze von der Spritzenlehre aufgenommen wird und ein Injektionsmittel in der Spritze mit einem vorgegeben Injektionsvolumen in das Auge injiziert wird.

Vorzugsweise ist die Spritzenlehre, insbesondere das Spritzensystem, zur Verwendung bei dem vorgenannten chirurgischen Eingriff an oder in dem Auge vorgesehen, wobei das vorgegebene Injektionsvolumen in Abhängigkeit von dem die Spritze aufnehmenden Verbindungsabschnitt der Spritzenlehre definiert wird. Das Spritzensystem kann sterilisiert und/oder für den Einmalgebrauch und/oder wiederverwendbar sein.

Vorzugsweise ist die Spritzenlehre, insbesondere das Spritzensystem, zur Verwendung bei einem der vorgenannten chirurgischen Eingriffen an oder in dem Auge vorgesehen, wobei das vorgegebene Injektionsvolumen in Abhängigkeit von der Ametropie oder der Emmetropie des Auges definiert wird. Dabei kann vorgesehen sein, dass das Injektionsvolumen bei einem myopen Auge größer als bei einem emmetropen Auge ist, und dass das Injektionsvolumen bei einem emmetropen Auge größer ist als bei einem hyperopen Auge ist. Insofern kann der Verbindungsabschnitt mit der größten Längserstreckung dem vordefinierten Injektionsvolumen für ein myopes Auge zugeordnet sein und der Verbindungsabschnitt mit der kleinsten Längserstreckung dem vordefinierten Injektionsvolumen für ein hyperopes Auge. Ein Verbindungsabschnitt kann einem emmetropen Auge zugeordnet und/oder mit einer "0" markiert sein. Ein Verbindungsabschnitt kann einem myopen Auge zugeordnet und/oder mit einem "+" oder "-" markiert sein. Ein Verbindungsabschnitt kann einem hyperopen Auge zugeordnet und entsprechend mit einem "-" oder "+" markiert sein.

Die Spritzenlehre, insbesondere das Spritzensystem, kann zur Verwendung bei einer intravitrealen operativen Medikamentenapplikation bestimmt sein, die üblicherweise auch als IVOM abgekürzt wird. Dabei kann vorgesehen sein, dass mittels Spritzen im Rahmen einer intravitrealen Operation bestimmte Arzneistoffe ins Auge eingebracht werden.

Die Spritzenlehre kann derart ausgebildet sein, dass maximal eine Spritze gleichzeitig von der Spritzenlehre aufnehmbar, insbesondere mit dieser verbindbar, ist. Entsprechend kann das Spritzensystem dazu ausgebildet sein, dass maximal eine Spritze gleichzeitig aufgenommen, insbesondere verbunden, ist.

Eine Verwendung des erfindungsgemäßen Spritzensystems sieht vorzugsweise vor, dass maximal eine Spritze gleichzeitig mit der Spritzenlehre verbunden wird.

Weitere Merkmale und Vorteile der Erfindung ergeben sich aus den Ansprüchen und aus der nachfolgenden Beschreibung, in der Ausführungsbeispiele der Erfindung unter Bezugnahme auf die Zeichnungen im Einzelnen erläutert sind. Dabei zeigen:
- Fig. 1: eine erfindungsgemäße Spritzenlehre einer perspektivischen Ansicht aus oben-proximal,
- Fig. 2: die Spritzenlehre gemäß Fig. 1 in einer weiteren perspektivischen Ansicht aus oben-proximal,
- Fig. 3: die Spritzenlehre gemäß Fig. 1 in einer Ansicht von oben,
- Fig. 4: die Spritzenlehre gemäß Fig. 1 in einer Ansicht aus distal,
- Fig. 5: die Spritzenlehre gemäß Fig. 1 in einer weiteren perspektivischen Ansicht von unten-proximal,
- Fig. 6: die Spritzenlehre gemäß Fig. 1 in einer weiteren perspektivischen Ansicht von unten-distal,
- Fig. 7: ein erfindungsgemäßes Spritzensystem mit der Spritzenlehre gemäß Fig. 1 und einer Spritze in einer perspektivischen Ansicht von schräg oben,
- Fig. 8: das Spritzensystem gemäß Fig. 7 in einer weiteren perspektivischen Ansicht von schräg unten und
- Fig. 9: das Spritzensystem gemäß Fig. 7 in einer Aufsicht.

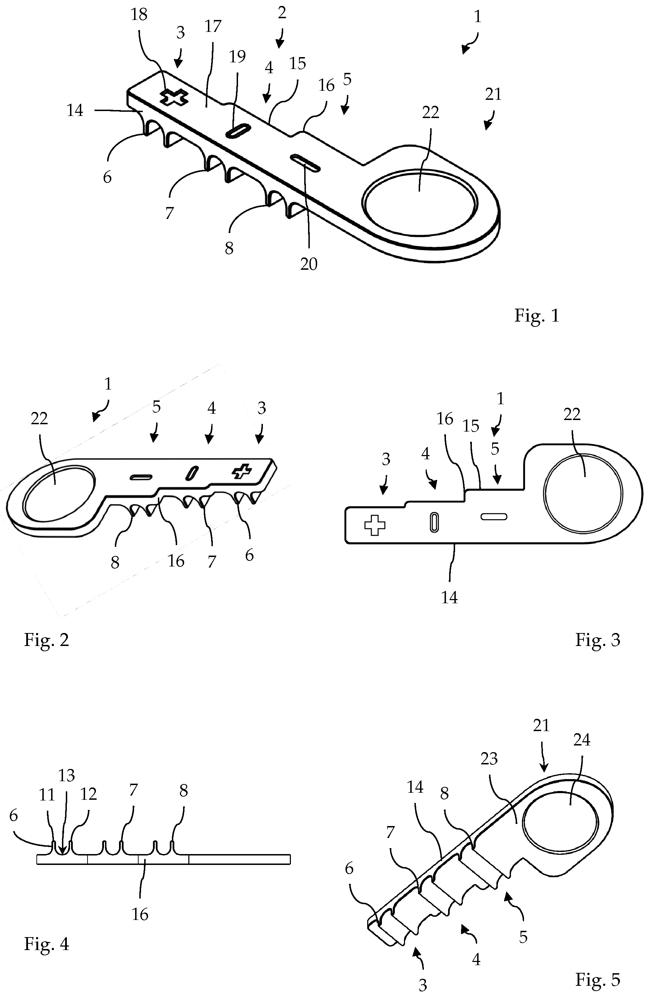

Fig. 1 zeigt eine erfindungsgemäße Spritzenlehre 1 in einer schematischen Ansicht aus oben-distal. Im Sinne der Erfindung weist eine distale Richtung zu dem Patienten und eine proximale Richtung zu dem Benutzer der Spritzenlehre 1. Eine Richtung von proximal nach distal oder umgekehrt weist in die axiale Richtung oder Längsrichtung.

Die Spritzenlehre 1 weist einen in Fig. 1 links angeordneten Verbindungsbereich 2 mit drei lateral nebeneinander angeordneten Verbindungsabschnitten 3, 4, 5 auf. Jeder Verbindungsabschnitt 3, 4, 5 ist mit einem nach unten weisenden Befestigungsmittel 6, 7, 8 zur Aufnahme eines Spritzenzylinders 9 einer Spritze 10 versehen, was weiter unten beschrieben ist. Das Befestigungsmittel 6, 7, 8 sind jeweils parallel zu der Längsrichtung der Spritzenlehre 1 ausgerichtet und weisen im gezeigten Ausführungsbeispiel zwei nebeneinander angeordnete und nach unten weisende Vorsprünge 11, 12 auf, zwischen denen sich ein halbkreisförmiger Zwischenbereich 13 erstreckt, so dass das Befestigungsmittel 6, 7, 8 insofern zumindest bereichsweise U-förmig ausgebildet ist. Die Befestigungsmittel 6, 7, 8 der drei Verbindungsabschnitte 3, 4, 5 sind im Wesentlichen identisch zueinander ausgebildet. Dies ist insbesondere in der Ansicht auf die Spritzenlehre 1 aus proximal gemäß Fig. 4 dargestellt.

Die distale Stirnseite 14 des Verbindungsbereichs 2 der Spritzenlehre 1 ist eben ausgebildet. Demgegenüber ist auf der proximalen Stirnseite 15 des Verbindungsbereichs 2 zwischen zwei zueinander benachbart angeordneten Verbindungsabschnitten 3, 4, 5 eine abgerundete Stufe 16 ausgebildet. Die drei Verbindungsabschnitte 3, 4, 5 weisen unterschiedliche Längsausdehnungen auf und sind dadurch von dem Benutzer leicht zu unterscheiden. Zwei Verbindungsabschnitte 3, 5 weisen jeweils eine auf der Oberseite 17 der Spritzenlehre 1 angeordnete nicht-numerische Information in Form einer Vertiefung 18, 20 auf und ein weiterer, mittiger Verbindungsabschnitt 4 weist eine auf der Oberseite 17 der Spritzenlehre 1 angeordnete numerische Information in Form einer Vertiefung 19 auf. Im gezeigten Ausführungsbeispiel weisen der Verbindungsabschnitt 3 mit der kürzesten Längsausdehnung ein "Plus"-Zeichen 18, der daneben angeordnete Verbindungsabschnitt 4 eine "0" 19 und der Verbindungsabschnitt 5 mit der größten Längsausdehnung ein "Minus"-Zeichen 20 auf. Die Längen der Verbindungsabschnitte 3, 4, 5 weisen einen linearen Zusammenhang auf und können mathematisch durch eine lineare Funktion beschrieben werden.

Die Spritzenlehre 1 ist zum Einsatz mit Spritzen 10 im Rahmen eines chirurgischen Eingriffs an oder in einem Auge ausgebildet, wobei ein vordefiniertes Volumen eines in der Spritze 10 befindlichen Injektionsmittels in Abhängigkeit von der Ametropie oder der Emmetropie des Auges definiert ist. Daher entspricht der Verbindungsabschnitt 3 mit der kürzesten Längsausdehnung jenem vordefinierten Injektionsvolumen, das in ein hyperopes, also weitsichtiges Auge zu injizieren ist, da weitsichtige Augen üblicherweise ein geringeres Volumen aufweisen als normalsichtige bzw. emmetrope Augen. Letzteren ist der mittlere Verbindungsbereich 4 zugeordnet, der dem Injektionsvolumen für emmetrope Augen zugeordnet ist. Der mit "Minus" versehene Verbindungsbereich 5 mit der größten Längsausdehnung ist einem myopen, also kurzsichtigen Auge zugeordnet und definiert die hierfür vorgesehene Injektionsvolumen.

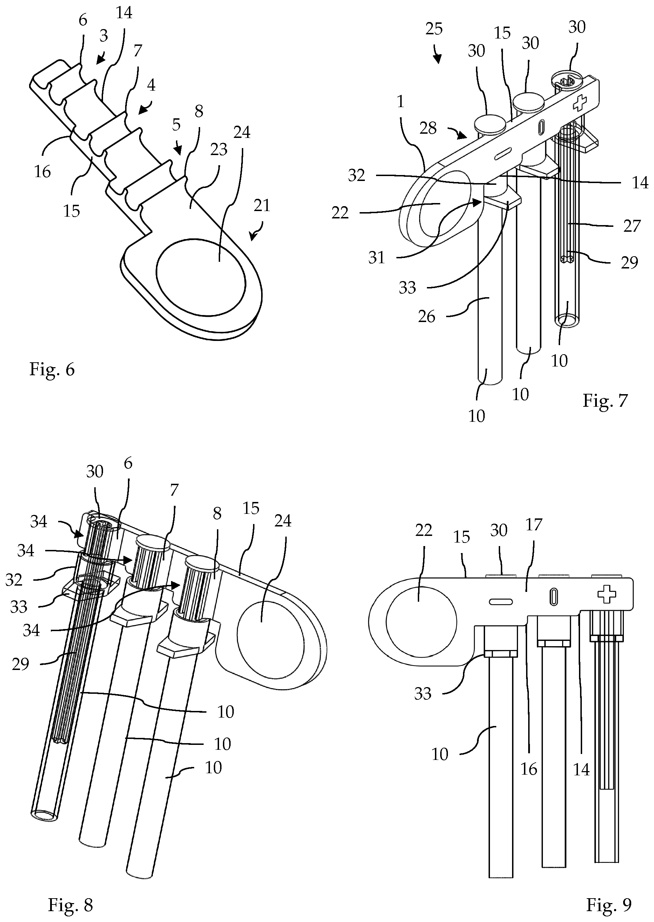

Benachbart zu dem Verbindungsbereich 2, insbesondere unmittelbar benachbart zu dem Verbindungsabschnitt 5 mit der größten Längsausdehnung, ist ein Griffbereich 21 der Spritzenlehre 1 ausgebildet, der eine kreisförmige Vertiefung 22 in Form einer Griffmulde aufweist, so dass die Spritzenlehre 1 leicht von dem Benutzer ergriffen und gehalten werden kann. Die Ansichten auf die Spritzenlehre 1 jeweils von schräg unten gemäß den Fig. 5 und 6 zeigen jeweils, dass auch die Unterseite 23 der Spritzenlehre 1 eine Vertiefung 24 im Griffbereich 21 aufweist.

Fig. 7 zeigt die erfindungsgemäße Spritzenlehre 1 gemäß Fig. 1 zusammen mit drei im Wesentlichen identisch ausgestalteten Spritzen 10, mithin ein erfindungsgemäßes Spritzensystem 25. Die Spritzen 10 weisen jeweils einen Spritzenzylinder 26 und einen innerhalb des Spritzenzylinders 26 längsverschieblich angeordneten Spritzenkolben 27 auf. Der Spritzenkolben 27 weist an einem proximalen Endbereich 28 einer Spritzenkolbenstange 29 eine laterale Verbreiterung 30 mit kreisförmiger Grundfläche auf, die dazu ausgebildet ist, in Anlage mit der proximalen Stirnseite 15 der Spritzenlehre 1 zu gelangen. Der Spritzenzylinder 26 weist an seinem proximalen Endbereich 31 ebenfalls eine laterale Verbreiterung 32 auf, die eine näherungsweise ringförmige Grundfläche aufweist und dazu ausgebildet ist, in Anlage mit der distalen Stirnseite 14 der Spritzenlehre 1 zu gelangen. Dieser Zustand ist in den Fig. 7 bis 9 gezeigt. Für eine bessere Handhabbarkeit der Spritze 10 kann der Spritzenzylinder 26 in dem proximalen Endbereich 31 zwei einander gegenüber angeordnete laterale Vorsprünge 33 aufweisen, mit denen die Spritze 10 leichter ergriffen werden kann. Die Vorsprünge 33 dienen als Widerlager für den Benutzer während der Längsbewegung des Spritzenkolbens 27 in dem Spritzenzylinder 26.

Aufgrund der unterschiedlich großen Längserstreckungen der Verbindungsabschnitte 3, 4, 5 sind die Spritzenkolben 27 unterschiedlich weit in den Spritzenzylindern 26 eingeschoben, was insbesondere durch die Ansicht aus unten-proximal gemäß Fig. 8 verdeutlicht ist. Demnach befinden sich unterschiedlich große Restvolumina in den gezeigten Spritzen 10, die maßgeblich von dem jeweils verbleibenden Hub 34 des Spritzenkolbens 27 und dem Durchmesser des Spritzenzylinders 26 abhängen.

Die Verwendung der erfindungsgemäßen Spritzenlehre 1 gestaltet sich wie folgt: Zunächst wird die zu verwendende Spritze 10 mit der erfindungsgemäßen Spritzenlehre 1 bereitgestellt. Dabei ist die Spritze 1 bereits mit dem zu injizierenden Injektionsmittel vorgefüllt. Unter Berücksichtigung der Ametropie bzw. der Emmetropie des Auges des Patienten, in das das Injektionsmittel zu injizieren ist, wählt der Benutzer den korrekten Verbindungsabschnitt 3, 4, 5 der Spritzenlehre 1 aus, so dass die Spritze 10 von dem diesem Verbindungsabschnitt 3, 4, 5 zugeordneten Befestigungsmittel 6, 7, 8 aufgenommen ist. Danach wird der Spritzenkolben 27 solange nach distal bewegt, bis er in Anlage mit der Spritzenlehre 1 gelangt. Das dabei abgegebene Injektionsvolumen wird entsorgt. Nun ist sichergegangen, dass ein vordefinierter Hub 34 der Spritze 1 verbleibt, der zusammen mit dem bekannten Durchmesser des Spritzenzylinders 26 dem gewählten, vordefinierten und in das Auge zu injizierenden Injektionsvolumen entspricht. Die Spritzenlehre 1 wird von der Spritze 10 entfernt und danach wird das Injektionsmittel mit dem vorgegebenen Injektionsvolumen in das Auge injiziert.

## Patentansprüche

1. Spritzenlehre (1) für eine Spritze (10) mit einem in einem Spritzenzylinder (26) längsverschieblich angeordneten Spritzenkolben (27) zum Einstellen eines vorgegebenen Injektionsvolumens, wobei die Spritzenlehre (1) einen Verbindungsbereich (2) mit mindestens zwei Verbindungsabschnitten (3, 4, 5) aufweist, wobei die Verbindungsabschnitte (3, 4, 5) jeweils ein Befestigungsmittel (6, 7, 8) zum Aufnehmen der Spritze (10) aufweisen, wobei sich die Befestigungsmittel (6, 7, 8) parallel zu der Längsrichtung der Spritze (10) erstrecken und wobei die Verbindungsabschnitte (3, 4, 5) senkrecht zu der Erstreckungsrichtung der Befestigungsmittel (6, 7, 8) versetzt zueinander angeordnet sind.

2. Spritzenlehre (1) nach Anspruch 1, **dadurch gekennzeichnet, dass** die Verbindungsabschnitte (3, 4, 5) zum Aufnehmen der Spritze (10) mit dem Spritzenkolben (27), insbesondere mit der Spritzenkolbenstange (29) und/oder proximal des Spritzenzylinders, ausgebildet sind.

3. Spritzenlehre (1) nach einem der Ansprüche 1 oder 2, **dadurch gekennzeichnet, dass** das Befestigungsmittel (6, 7, 8) zwei zueinander beabstandet angeordnete und/oder parallel zueinander ausgerichtete Vorsprünge (11, 12) aufweist.

4. Spritzenlehre (1) nach einem der Ansprüche 1 bis 3, **dadurch gekennzeichnet, dass** jedem Verbindungsabschnitt (3, 4, 5) mindestens eine insbesondere nicht-numerische Information (18, 19, 20) auf der Oberseite (17) der Spritzenlehre (1) zugeordnet ist.

5. Spritzenlehre (1) nach einem der Ansprüche 1 bis 4, **dadurch gekennzeichnet, dass** mindestens drei, insbesondere genau drei, Verbindungsabschnitte (3, 4, 5) ausgebildet sind, die insbesondere sämtlich parallel zueinander ausgerichtet sind und/oder die insbesondere verschiedene Längen aufweisen.

6. Spritzenlehre (1) nach einem der Ansprüche 1 bis 5, **dadurch gekennzeichnet, dass** zwischen zwei Verbindungsabschnitten (3, 4, 5), insbesondere zwischen unmittelbar zueinander benachbarten Verbindungsabschnitten (3, 4, 5), ein Übergang mit einer insbesondere abgerundeten Stufe (16) ausgebildet ist.

7. Spritzenlehre (1) nach einem der Ansprüche 1 bis 6, **dadurch gekennzeichnet, dass** benachbart zu dem Verbindungsbereich (2) ein Griffbereich (21) ausgebildet ist, der insbesondere benachbart zu dem Verbindungsabschnitt (5) mit der größten Längserstreckung, insbesondere unmittelbar benachbart daneben angeordnet ist.

8. Spritzensystem (25) mit einer Spritze (10) und eine mit der Spritze (10) verbindbare, insbesondere mit der Spritze verbundene, Spritzenlehre (1) nach einem der Ansprüche 1 bis 7.

9. Spritzenlehre (1) nach einem der Ansprüche 1 bis 7, insbesondere Spritzensystem (25) nach Anspruch 8, zur Verwendung bei einem chirurgischen Eingriff an oder in einem Auge, wobei die Spritzenlehre (1) mit der Spritze (10) verbunden wird und ein Injektionsmittel in der Spritze (10) mit einem vorgegebenen Injektionsvolumen in das Augen injiziert wird.

10. Spritzenlehre (1) nach einem der Ansprüche 1 bis 7, insbesondere Spritzensystem (25) nach Anspruch 8, zur Verwendung bei einem chirurgischen Eingriff an oder in einem Auge nach Anspruch 9, wobei das vorgegebene Injektionsvolumen in Abhängigkeit von dem mit der Spritze (10) verbundenen Verbindungsabschnitt der Spritzenlehre (1) definiert wird.

11. Spritzenlehre (1) nach einem der Ansprüche 1 bis 7, insbesondere Spritzensystem (25) nach Anspruch 8, zur Verwendung bei einem chirurgischen Eingriff an oder in einem Auge nach Anspruch 9 oder 10, wobei das vorgegebene Injektionsvolumen in Abhängigkeit von der Ametropie oder der Emmetropie des Auges definiert ist.
